(19) Europäisches Patentamt
European Patent Office
Office européen des brevets

(11) Publication number: 0 184 035
B1

(12) **EUROPEAN PATENT SPECIFICATION**

(45) Date of publication of patent specification: **25.07.90**

(51) Int. Cl.$^5$: **C 07 D 215/56** // A61K31/47

(21) Application number: **85114373.5**

(22) Date of filing: **12.11.85**

# (54) Quinolonecarboxylic acid derivatives and process for their preparation.

(30) Priority: **13.11.84 JP 239124/84**
**30.10.85 JP 243553/85**

(43) Date of publication of application:
**11.06.86 Bulletin 86/24**

(45) Publication of the grant of the patent:
**25.07.90 Bulletin 90/30**

(84) Designated Contracting States:
**BE CH DE FR GB IT LI NL SE**

(56) References cited:
**EP-A-0 106 489**

(73) Proprietor: **KYORIN PHARMACEUTICAL CO., LTD.**
**No. 5, Kanda Surugadai 2-chome**
**Chiyoda-ku Tokyo (JP)**

(72) Inventor: **Irikura, Tsutomu**
**No. 10-28, Ooizumigakuen-cho 7-chome**
**Nerima-ku Tokyo (JP)**
Inventor: **Suzue, Seigo**
**No. 13-4, Aoba 4-chome**
**Kuki-shi Saitama-ken (JP)**
Inventor: **Hirai, Keiji**
**No. 1-2-512, Aoba 1-chome**
**Kuki-shi Saitama-ken (JP)**
Inventor: **Ishizaki, Takayoshi**
**No. 9-6, Sakurada 4-chome Washimiya-machi**
**Kitakatwushika-gun Saitama-ken (JP)**

(74) Representative: **TER MEER - MÜLLER - STEINMEISTER & PARTNER**
**Mauerkircherstrasse 45**
**D-8000 München 80 (DE)**

**EP 0 184 035 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to quinolonecarboxylic acid derivatives and a method for their preparation.

The EP—A—0 106 489 describes naphthyridine-, quinoline- and benzoxazinecarboxylic acid derivatives and processes for their manufacture, formulation and use of antibacterial compounds in mammals.

The present invention is concerned with certain novel quinolonecarboxylic acid derivatives of the following formula (I)

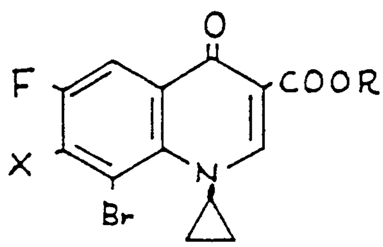

(I)

wherein R is a hydrogen atom or an ethyl group and X is a halogen atom, which are useful intermediates for the synthesis of a medicine, especially antibacterial agent, and with a process for their preparation.

Compounds of this type, which havea bromine atom at 8 position can not find in the prior art.

We have found that the introduction of a bromine atom to the 8 position in the antibacterial quinolonecarboxylic acid derivatives gives rise to enhance their activity.

Thus, 8-bromo-cyclopropyl-6-fluoro-1,4-dihydro-7-substituted amino-4-oxo-3-quinolinecarboxylic acid derivatives were found to be useful antibacterial agents.

The compounds of the present invention are used as intermediates for the synthesis of the agents.

The compounds of the formula (I) are synthesized as below.

The compound of the formula (II),

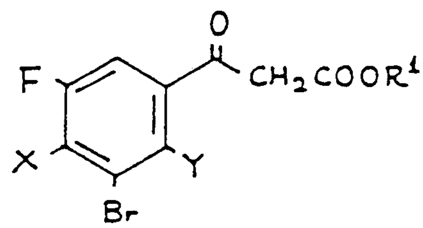

(II)

wherein $R^1$ is an ethyl group, X and Y are each independently a halogen atom, is reacted with an equimolar or excess amount of orthoformic ester in acetic anhydride (1 to 20-fold volume per the total volume of the other reagent(s) at room temperature to 200°C, preferably 100 to 150°C for several hours to give the compound of the formula (III),

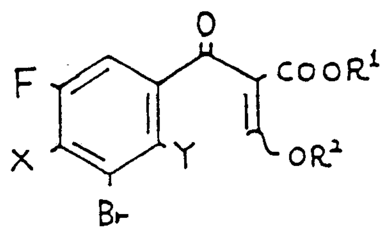

(III)

wherein $R^2$ is an ethyl group and $R^1$, X and Y have the above-stated meanings.

The compound of the formula (III) is converted by treating with cyclopropylamine (an equimolar or excess amount) in a solvent such as ethanol to the compound of the formula (IV),

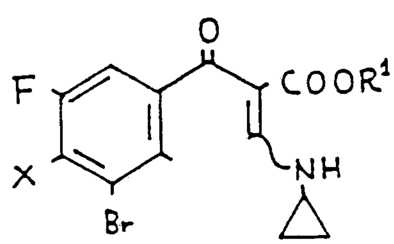

(IV)

wherein $R^1$, X and Y have the above-stated meanings.

The compound of the formula (IV) is treated with an appropriate base such as sodium fluoride, potassium fluoride or sodium hydride in a solvent such as dioxane, alcohol, dimethylformamide, dimethyl sulfoxide and sulfolane at 0 to 200°C, preferably 50 to 150°C for one or several hours to give the compound of the formula (I'),

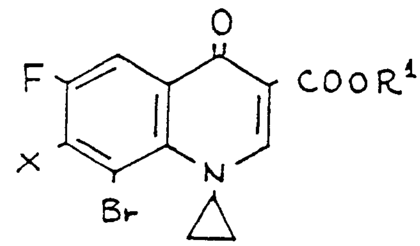

(I')

wherein $R^1$ and X have the above-stated meanings.

The compound of the formula (I') can be hydrolyzed by the usual acid or alkali hydrolysis to the compound of the formula (I''),

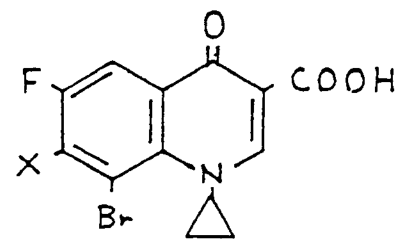

(I")

wherein X has the above-stated meaning.

The starting compound of the formula (II) is also new and obtained, for example, from 3-chloro-4-fluoroaniline via several steps as illustrated in the following chart and in example.

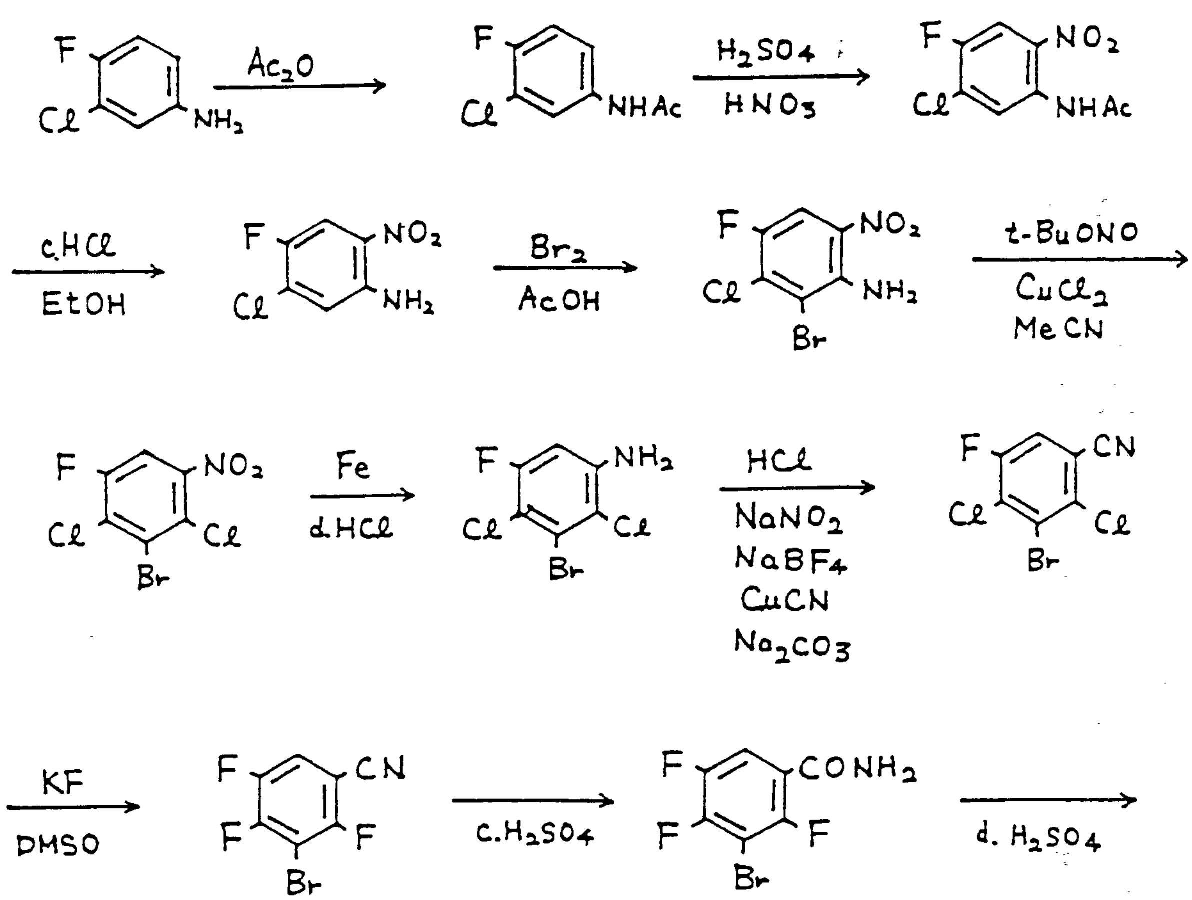

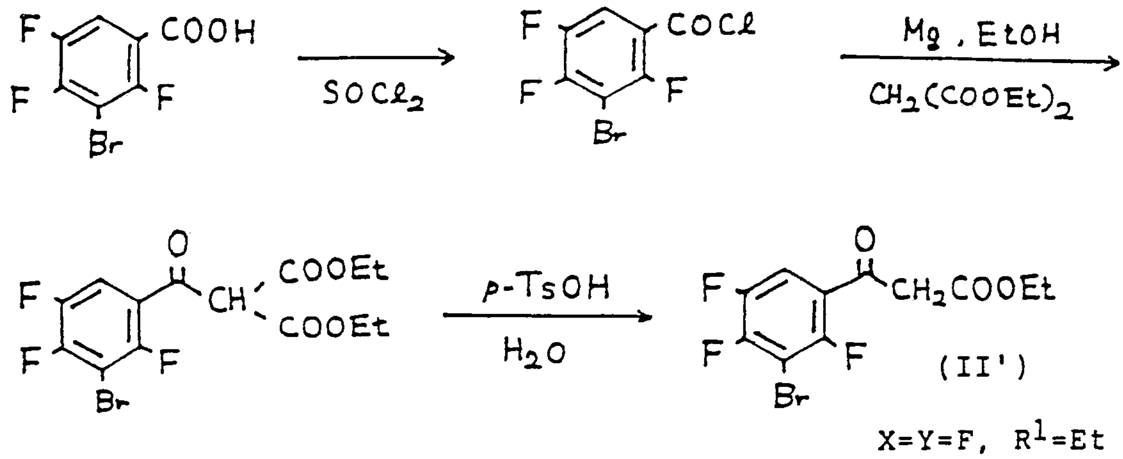


(II')

X=Y=F, $R^1$=Et

The following example will further illustrate the invention without, however, limiting it thereto.

Example 1

N-(3-Chloro-4-fluorophenyl)acetamide

Acetic anhydride (200 ml) was slowly added to 3-chloro-4-fluoroaniline (100 g). After allowed to stand for 30 minutes, the reaction mixture was poured into water (1 liter). The resulting precipitate was collected by filtration and recrystallized from aqueous ethanol to give the title compound (119.4 g), mp 118—119°C.

Example 2

N-(3-Chloro-4-fluoro-6-nitrophenyl)acetamide

Concentrated nitric acid (d 1.42, 154 ml) was added dropwise to a solution of N-(3-chloro-4-fluorophenyl)acetamide (55 g) in concentrated sulfuric acid (165 ml) at 5—10°C during an hour with stirring in an ice-salt bath. After stirring for an hour at the same temperature, the reaction mixture was poured into ice water. The resulting precipitate was collected by filtration, sufficiently washed with water and recrystallized from acetonitrile to give the title compound (48.9 g) as yellow needles, mp 114—115°C.

Example 3

3-Chloro-4-fluoro-6-nitroaniline

A solution of N-(3-chloro-4-fluoro-6-nitrophenyl)acetamide (30 g) in concentrated hydrochloric acid (50 ml) and ethanol (200 ml) was refluxed for 2.5 hours. Ice water (300 ml) was added to the reaction mixture and the resulting precipitate was collected by filtration, washed with water and dried to give the title compound (24.9 g) as yellow needles, mp 149.5—150°C.

Analysis (%) for $C_6H_4ClFN_2O_2$, Calcd. (Found): C, 37.82 (37.85); H, 2.11 (2.03); N, 14.70 (14.80).

Example 4

2-Bromo-3-chloro-4-fluoro-6-nitroaniline

Bromine (339 g) was added into a solution of 3-chloro-4-fluoro-6-nitroaniline (200.3 g) in acetic acid (1.5 liter) during a period of 80 minutes at 50°C under stirring and stirred for further 2 hours. The reaction mixture was poured into ice water (3 liter) and the resulting precipitate was collected by filtration, washed with water and added to a mixture of concentrated hydrochloric acid (300 ml) and ethanol (1.2 liter). The mixture was refluxed for 8.5 hrs. After cooling, the precipitate was collected by filtration and washed with water and dried. The title compound thus obtained weighed 235.6 g as yellow needles, mp 146—147°C.

Example 5

3-Bromo-2,4-dichloro-5-fluoronitrobenzene

Tert-butylnitrite (135.2 g) was added to a mixture of anhydrous cupric chloride (147 g) and 2-bromo-3-chloro-4-fluoro-6-nitroaniline (235.6 g) in anhydrous acetonitrile (1.5 liter) at 60°C during 70 minutes. The reaction mixture was poured into ice-chilled diluted hydrochloric acid (1.5 liter) and extracted with benzene. The organic layer was successively washed with ice-chilled diluted hydrochloric acid and water saturated with sodium chloride, dried over anhydrous sodium sulfate and concentrated. The resulting residue was purified by distillation to give the title compound (218.8 g), bp 78—117°C/2 mmHg. The oil was crystallized from methanol to give yellow prisms, mp 65.5—67.5°C.

Example 6

3-Bromo-2,4-dichloro-5-fluoroaniline

Concentrated hydrochloric acid (18 ml) was slowly added to a suspension of iron powder (135.4 g) in water (140 ml), with vigorous stirring at 50—60°C. After ethanol (350 ml) was mixed, 3-bromo-2,4-dichloro-5-fluoronitrobenzene (218.8 g) was added portionwise to the suspension at 52—76°C during an hour. After stirring for 75 minutes at the same temperature, the hot reaction mixture was filtered after adding benzene (500 ml) and the insoluble material was successively washed with hot ethanol (100 ml) and benzene (200 ml). The filtrate and washings were combined. The organic layers were washed with water saturated with sodium chloride, dried over anhydrous sodium sulfate and then concentrated. The resulting residue was recrystallized from ethanol-water to give the title compound (141.6 g) as light brown needles, mp 126—129.5°C.

Example 7

3-Bromo-2,4-dichloro-5-fluorobenzonitrile

Sodium nitrite (56.6 g) in water (120 ml) was added to a suspension of 3-bromo-2,4-dichloro-5-fluoroaniline (141.6 g) in concentrated hydrochloric acid (900 ml) with vigorous stirring at −2~0°C for 40 minutes. After stirring for 30 minutes, the mixture was poured into ice water (700 ml) containing sodium tetrafluoroborate (180 g), stirred vigrously for 20 minutes and then allowed to stand for 15 minutes in an ice bath. The resulting precipitate was collected by filtration and washed with chilled water. The wet crude tetrafluoroborate thus obtained weighed 270.8 g. The borate was added portionwise during 45 minutes to a solution of cuprous cyanide (98 g), potassium cyanide (142.4 g) and sodium carbonate (29 g) in water (800 ml) with vigorous stirring at 9—10°C. After the mixture was stirred for 2 hours at room temperature, benzene (700 ml) and potassium cyanide (71 g) were added to the suspension and then the mixture was stirred for 30 minutes. The insoluble material was collected by filtration, and washed with benzene (300 ml × 2).

The filtrate and washings were combined and washed five times with water saturated with sodium chloride, dried over anhydrous sodium sulfate and then concentrated. The resulting residue was recrystallized from ethanol to give the title compound (75.5 g) as red brown prisms, mp 110.5—112.5°C.

Example 8

3-Bromo-2,4,5-trifluorobenzonitrile

3-Bromo-2,4-dichloro-fluorobenzonitrile (68,4 g) was added to a solution of potassium fluoride (123 g) in dimethyl sulfoxide (400 ml) with stirring at 133°C and then the mixture was stirred for 5 hours and 20 minutes at 130°C. After cooling, the reaction mixture was poured into ice water (1 liter) and extracted with benzene. The organic layer was washed with water saturated with sodium chloride, dried over anhydrous sodium sulfate and distilled to give the title compound (15.7 g) as colorless oil, bp 82.5°C/13 mmHg — 80.0°C/1 mmHg.

Example 9

3-Bromo-2,4,5-trifluorobenzoic acid

A mixture of 3-bromo-2,4,5-trifluorobenzonitrile (13.9 g) in concentrated sulfuric acid (8 ml) was heated for 20 minutes on an oil bath (100°C), poured into ice water (350 ml). The resulting precipitate was collected by filtration and washed with water. The filtrate and washings were extracted 3 times with dichloromethane. The dichloromethane layer was washed with water saturated with sodium chloride, dried over anhydrous sodium sulfate and concentrated to give the residue. The combined mixture of the precipitate obtained previously and the residue was purified by silica gel chromatography eluting with dichloromethane → dichloromethane:methanol (10:1) to give 3-bromo-2,4,5-trifluorobenzamide (8.7 g).

A mixture of 3-bromo-2,4,5-trifluorobenzamide (8.7 g) and 18N-sulfuric acid (50 ml) was stirred at 100°C for 4 hours, and then poured into ice water (200 ml). The resulting precipitate was collected by filtration and recrystallized from dichloromethane-n-hexane to give the title compound (6.9 g), mp 125—127°C.

Example 10

3-Bromo-2,4,5-trifluorobenzoyl chloride

A solution of the 3-bromo-2,4,5-trifluorobenzoic acid (2.5 g) in thionyl chloride (10 ml) was refluxed for 2.5 hours, and then concentrated. The resulting residue was purified by distillation through Widmer fractionating column to give the title compound (2.3 g), bp 98—102°C/18 mmHg.

Example 11

Diethyl 3-bromo-2,4,5-trifluorobenzoylmalonate

Magnesium turnings (0.22 g) and carbon tetrachloride (0.1 ml) was added to absolute ethanol (1.5 ml). To the stirring suspension was added dropwise a solution of diethyl malonate (1.4 g) and absolute ethanol (2 ml) in toluene (6 ml) during 25 minutes at 50—60°C. The mixture was stirred for 40 minutes, and then cooled. A solution of 3-bromo-2,4,5-trifluorobenzoyl chloride (2.27 g) in anhydrous toluene (3 ml) was added dropwise to the solution at −8~−4.5°C during 28 minutes. The mixture was stirred for 2 hours and then mixed with ice-chilled diluted sulfuric acid. The resulting organic layer was collected and the water layer was extracted with toluene (6 ml × 4). The combined organic layer was washed with water, dried over anhydrous sodium sulfate and then concentrated to give the title compound (3.25 g) as pale yellow oil.

### Example 12

Ethyl 3-bromo-2,4,5-trifluorobenzoylacetate

P-Toluenesulfonic acid (4 mg) was added to an emulsion of diethyl 3-bromo-2,4,5-trifluorobenzoylmalonate (3.25 g) in water (4 ml) and refluxed for 3 hours with vigorous stirring. After cooling, the reaction mixture was extracted with dichloromethane (8 ml × 4). The organic layer was washed with water saturated with sodium chloride, dried over anhydrous sodium sulfate and concentrated. The residue was recrystallized from dichloromethane-n-hexane to give the title compound (1.51 g), mp 85—88°C.

### Example 13

Ethyl 2-(3-bromo-2,4,5-trifluorobenzoyl)-3-ethoxyacrylate

A mixture of ethyl 3-bromo-2,4,5-trifluorobenzoylacetate (1.5 g), ethyl orthoformate (1.0 g) and acetic anhydride (1.2 g) was stirred at 130°C for 4.5 hours and then concentrated to give the title compound (1.75 g) as yellow oil.

### Example 14

Ethyl 2-(3-bromo-2,4,5-trifluorobenzoyl)-3-cyclopropylaminoacrylate

A solution of cyclopropylamine (0.32 g) in absolute ethanol (2 ml) was added to a solution of ethyl 2-(3-bromo-2,4,5-trifluorobenzoyl)-3-ethoxyacrylate (1.75 g) in absolute ethanol (5 ml) under ice-cooling during 30 minutes. The mixture was stirred at 5—20°C for 2.5 hours and concentrated. The residue was recrystallized from petroleum ether to give the title compound (1.36 g), mp 74—76°C.

### Example 15

Ethyl 8-bromo-1-cyclopropyl-6,7-difluoro-1,4-dihydro-4-oxo-3-quinolinecarboxylate

Sodium fluoride (0.23 g) was added to a solution of ethyl 2-(3-bromo-2,4,5-trifluorobenzoyl)-3-cyclopropylaminoacrylate (1.35 g) in anhydrous dimethylformamide (5 ml). The mixture was stirred for 97—108°C for 7.5 hours, and then poured into ice water (50 ml) and the resulting precipitate was collected by filtration, washed with water and recrystallized from dichloromethane-n-hexane to give the title compound (1.05 g), mp 163.5—168°C as colorless prisms.

NMR (δ in $CDCl_3$), 1.0—1.4 (4H, m, 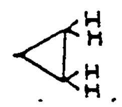 ), 1.40 (3H, t, J=7.3 Hz, $—CH_2CH_3$), 4.1—4.3 (1H, m, 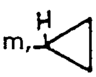 4.39 (2H, q, J=7.2 Hz, $CH_2CH_3$), 8.26 (1H, dd, J=9.7, 8.4 Hz, 5-H), 8.68 (1H, s, 2-H).

IR (KBr, $cm^{-1}$), 1730, (COO), 1600 (CO), 1450, 1310, 1270, 1230, 1200, 1170, 1070, 1030, 860, 800.

### Example 16

8-Bromo-1-cyclopropyl-6,7-difluoro-1,4-dihydro-4-oxo-3-quinolinecarboxylic acid

A mixture of ethyl 8-bromo-1-cyclopropyl-6,7-difluoro-1,4-dihydro-4-oxo-3-quinolinecarboxylate (1.0 g), acetic acid (4 ml), water (3 ml) and concentrated sulfuric acid (0.5 ml) was heated on an oil bath (90—100°C) for an hour under stirring, then for an hour at room temperature and poured into ice water (20 ml). The resulting precipitate was collected by filtration and washed with water to give the title compound (0.82 g), mp 224—225.5°C.

NMR (δ in $CDCl_3$), 1.0—1.5 (4H, m, 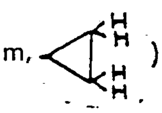 ), 4.3—4.5 (1H, m, 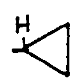 ) 8.30 (1H, dd, J=9.2, 8.4 Hz, 5-H), 8.96 (1H, s, 2-H), 13.97 (1H, s—br, —COOH).

IR (KBr, $cm^{-1}$), 2700 (COOH), 1720 (COO), 1610 (CO), 1560, 1450, 1320, 1310, 1260, 1090, 1070, 1040, 1020, 880, 870, 850, 830, 810, 800, 730.

## Claims

1. A compound of the formula (I):

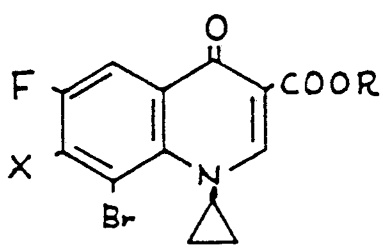

(I)

wherein R is a hydrogen atom or an ethyl group and X is a halogen atom.

2. A process for the preparation of a compound of the formula (I) specified in claim 1, where R = ethyl which comprises condensing a compound of the formula (II),

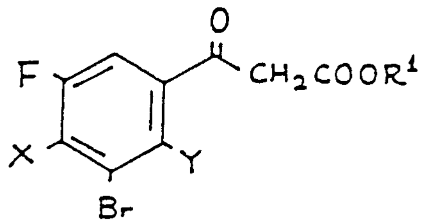

(II)

wherein $R^1$ is an ethyl group and X and Y are each independently a halogen atom; with trialkyl orthoformate to give the compound of the formula (III),

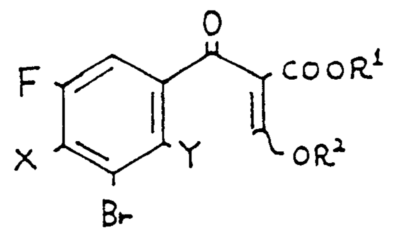

(III)

wherein $R^2$ is an ethyl group and $R^1$ and X have the above-stated meanings, reacting the compound of the formula (III) with cyclopropylamine to form the compound of the formula (IV),

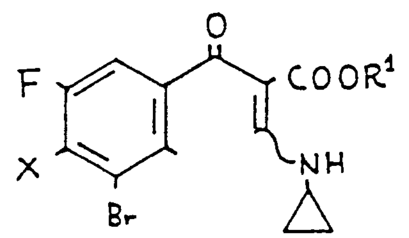

(IV)

wherein $R^1$, X and Y have the above-stated meanings, and cyclizing the compound of the formula (IV),

3. A process for the preparation of a compound of the formula (I''),

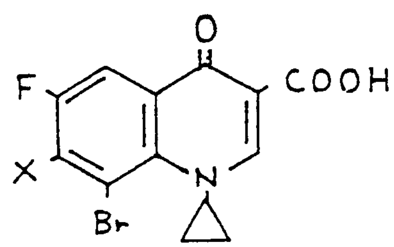

(I")

wherein X is a halogen atom, which comprises hydrolyzing a compound of the formula (I'),

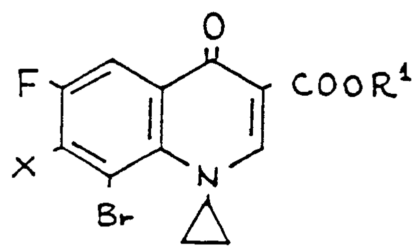

(I')

wherein $R^1$ is an ethyl group and X has the above-stated meaning.

**Patentansprüche**

1. Verbindung der Formel (I),

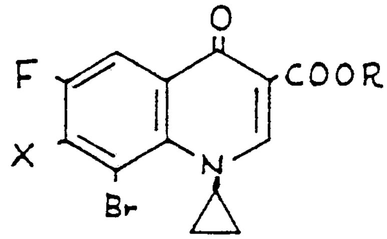

(I)

worin R ein Wasserstoffatom oder eine Ethylgruppe und X ein Halogenatom darstellen.

2. Verfahren zur Herstellung einer Verbindung der Formel (I) nach Anspruch 1, worin R eine Ethylgruppe bedeutet, welches darin besteht, eine Verbindung der Formel (II),

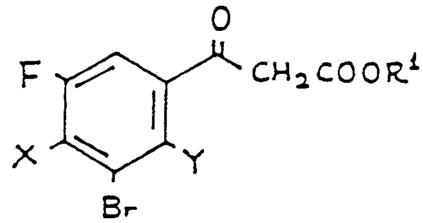

(II)

worin $R^1$ eine Ethylgruppe und X und Y unabhängig voneinander ein Halogenatom darstellen, mit einem Trialkyl-orthoformiat zur Darstellung einer Verbindung der Formel (III),

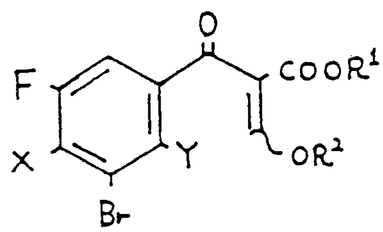

(III)

worin $R^2$ eine Ethylgruppe bedeutet und $R^1$ X die oben angegebenen Bedeutungen besitzen, zu kondensieren, die Verbindung der Formel (III) mit Cyclopropylamin zur Bildung einer Verbindung der Formel (IV),

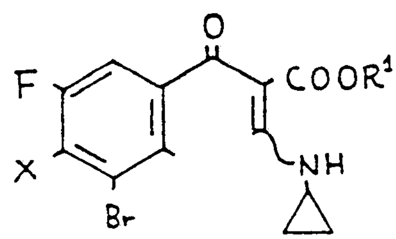

(IV)

worin $R^1$, X und Y die oben angegebenen Bedeutungen besitzen, umzusetzen und die Verbindung der Formel (IV) zu cyclisieren.

3. Verfahren zur Herstellung einer Verbindung der Formel (I''),

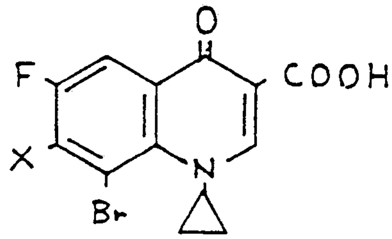

(I")

worin X ein Halogenatom bedeutet, welches darin besteht, eine Verbindung der formel (I'),

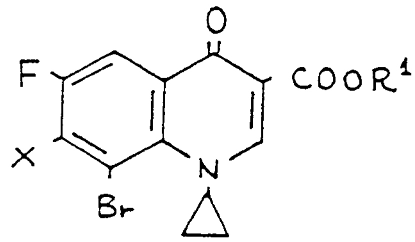

(I')

worin $R^1$ eine Ethylgruppe darstellt und X die oben angegebenen Bedeutungen besitzt zu hydrolysieren.

**Revendications**

1. Composé de formule (I):

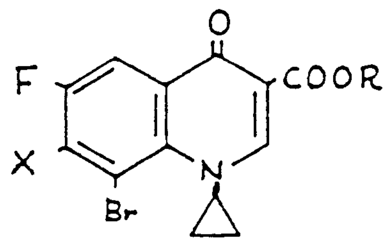

(I)

dans laquelle R est un atome d'hydrogène ou un groupe éthyle et X est un atome d'halogène.

2. Procédé pour la préparation d'un composé de formule (I) specifié dans la revendication 1 où R = éthyle, qui consiste à condenser un composé de formule (II):

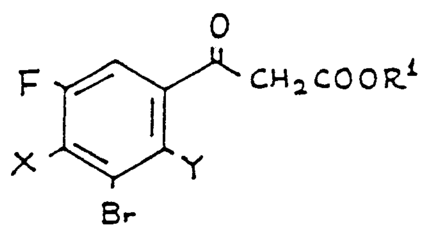

(II)

dans laquelle $R^1$ est un groupe éthyle, et X et Y sont chacune indépendamment un atome d'halogène, avec un orthoformiate de trialkyle pour obtenir le composé de formule (III):

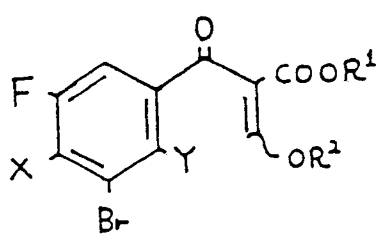

(III)

dans laquelle $R^2$ est un groupe éthyle, et $R^1$ et X ont les définitions données ci-dessus, à faire réagir le composé de formule (III) avec la cyclopropylamine pour former le composé de formule (IV):

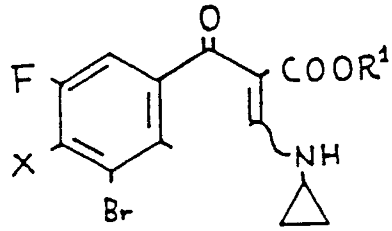

(IV)

dans laquelle $R^1$, X et Y ont les définitions données ci-dessus, et à cycliser le composé de formule (IV).

3. Procédé pour la préparation d'un composé de formule (I''):

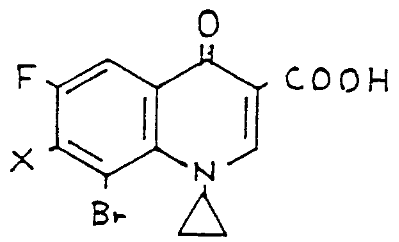

(I")

dans laquelle X est un atome d'halogène, qui consiste à hydrolyser un composé de formule (I'):

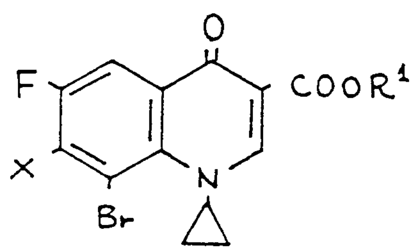

(I')

dans laquelle $R^1$ est un groupe éthyle et X a la définition donnée ci-dessus.